(19) Europäisches Patentamt
European Patent Office
Office européen des brevets

(11) EP 1 527 048 B1

(12) **FASCICULE DE BREVET EUROPEEN**

(45) Date de publication et mention de la délivrance du brevet:
**09.01.2008 Bulletin 2008/02**

(21) Numéro de dépôt: **03755635.4**

(22) Date de dépôt: **25.07.2003**

(51) Int Cl.:
***C07D 211/26*** *(2006.01)* ***A61K 31/4402*** *(2006.01)*
***A61P 25/00*** *(2006.01)*

(86) Numéro de dépôt international:
**PCT/FR2003/002356**

(87) Numéro de publication internationale:
**WO 2004/013101 (12.02.2004 Gazette 2004/07)**

(54) **DERIVES DE N-[PHENYL(PIPERIDIN-2-YL)METHYL]BENZAMIDE, LEUR PREPARATION ET LEUR APPLICATION EN THERAPEUTIQUE**

N-[PHENYL(PIPERIDIN-2-YL)METHYL]BENZAMID-DERIVATE, VERFAHREN ZU DEREN HERSTELLUNG UND IHRE THERAPEUTISCHE VERWENDUNG

N-[PHENYL(PIPERIDIN-2-YL)METHYL]BENZAMIDE DERIVATIVES, PREPARATION THEREOF, AND USE THEREOF IN THERAPY

(84) Etats contractants désignés:
**AT BE BG CH CY CZ DE DK EE ES FI FR GB GR HU IE IT LI LU MC NL PT RO SE SI SK TR**
Etats d'extension désignés:
**AL LT LV MK**

(30) Priorité: **29.07.2002 FR 0209588**

(43) Date de publication de la demande:
**04.05.2005 Bulletin 2005/18**

(73) Titulaire: **Sanofi-Aventis**
**75013 Paris (FR)**

(72) Inventeurs:
- **DARGAZANLI, Gihad**
  **F-94230 Cachan (FR)**
- **ESTENNE-BOUHTOU, Geneviève**
  **F-94550 Chevilly-Larue (FR)**
- **MARABOUT, Benoît**
  **F-91300 Massy (FR)**
- **ROGER, Pierre**
  **F-78180 Montigny-le-Bretonneux (FR)**
- **SEVRIN, Mireille**
  **F-75014 Paris (FR)**

(74) Mandataire: **Ludwig, Jacques et al**
**Sanofi-Aventis**
**174 avenue de France**
**75013 Paris (FR)**

(56) Documents cités:
**WO-A-99/45011** **US-A- 5 254 569**

- **O. FROELICH ET AL.: "Asymmetric synthesis. 39. Synthesis of 2-(1-aminoalkyl)piperidines via 2-cyano-6-phenyl oxazolopiperidine" J. ORG. CHEM., vol. 61, 1996, pages 6700-6705, XP002235079**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 527 048 B1

Printed by Jouve, 75001 PARIS (FR)

## Description

Les composés de l'invention répondent à la formule générale (I)

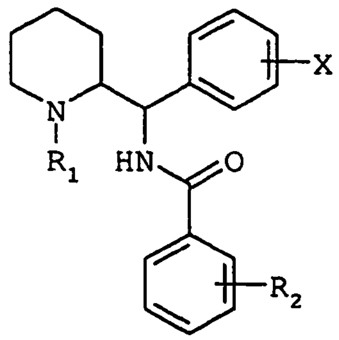

(I)

dans laquelle

$R_1$ représente soit un atome d'hydrogène, soit un groupe $(C_1-C_7)$alkyle linéaire ou ramifié éventuellement substitué par un ou plusieurs atomes de fluor, soit un groupe $(C_3-C_7)$cycloalkyl$(C_1-C_3)$alkyle, soit un groupe phényl$(C_1-C_3)$ alkyle éventuellement substitué par un ou deux groupes méthoxy, soit un groupe $(C_2-C_4)$alcényle, soit un groupe $(C_2-C_4)$alcynyle,

X représente un atome d'hydrogène ou un ou plusieurs substituants choisis parmi les atomes d'halogènes et les groupes trifluorométhyle, $(C_1-C_4)$alkyle linéaire ou ramifié et $(C_1-C_4)$ alcoxy,

$R_2$ représente un ou plusieurs substituants choisis parmi les atomes d'halogènes, parmi les groupe de formule générale $OR_3$ dans laquelle $R_3$ représente un atome d'hydrogène, un groupe $(C_1-C_4)$ alkyle, un groupe phényl $(C_1-C_3)$alkyle, ou un groupe de formule générale $(CH_2)_n$-$NR_4R_5$ dans laquelle n représente le nombre 2, 3 ou 4 et $R_4$ et $R_5$ représentent chacun, indépendamment l'un de l'autre, un atome d'hydrogène ou un groupe $(C_1-C_4)$ alkyle ou forment, avec l'atome d'azote qui les porte, un cycle pyrrolidine, pipéridine ou morpholine, et parmi les groupes amino de formule générale $NR_6R_7$ dans laquelle $R_6$ et $R_7$ représentent chacun, indépendamment l'un de l'autre, un atome d'hydrogène ou un groupe $(C_1-C_4)$alkyle, un groupe phényle ou un groupe phényl$(C_1-C_3)$alkyle, ou forment, avec l'atome d'azote qui les porte, un cycle pyrrolidine, pipéridine ou morpholine.

Les composés de formule générale (I) peuvent exister sous forme du racémate thréo (1*R*,2*R* ; 1*S*,2*S*) ou sous forme d'énantiomères (1*R*,2*R*) ou (1*S*,2*S*); ils peuvent exister à l'état de bases libres ou de sels d'addition à des acides.

Des composés de structure analogue à celle des composés de l'invention sont décrits dans le brevet US-5254569 comme analgésiques, diurétiques, anticonvulsivants, anesthésiques, sédatifs, cérébroprotecteurs, par un, mécanisme d'action sur les récepteurs opiacés. D'autres composés de structure analogue sont décrits dans la demande de brevet EP-0499995 comme antagonistes 5-$HT_3$ utiles dans le traitement des désordres psychotiques, des maladies neurologiques, des symptômes gastriques, des nausées et des vomissements. WO 99/45011 décrit certains α,α-diphényl-1-pipéridinebutanamides comme inhibiteurs des transporteurs de la glycine.

Les composés préférés de l'invention sont dépourvus d'activité sur les récepteurs opiacés ou 5-HT3 et présentent une activité particulière comme inhibiteurs spécifiques des transporteurs de la glycine glyt1 et/ou glyt2.

Les composés préférés comme inhibiteurs du transporteur glyt1 sont de configuration (1*S*,2*S*) avec $R_2$ représentant un ou plusieurs atomes d'halogènes, alors que les composés préférés comme inhibiteurs du transporteur glyt2 sont de configuration (1*R*,2*R*) avec $R_2$ représentant un ou plusieurs atomes d'halogènes et un groupe amino de formule générale $NR_6R_7$.

Les composés de formule générale (I) dans laquelle $R_1$ est différent d'un atome d'hydrogène peuvent être préparés par un procédé illustré par le schéma 1 qui suit.

On effectue un couplage d'une diamine de formule générale (II), dans laquelle $R_1$ et X sont tels que définis ci-dessus (avec $R_1$ différent d'un atome d'hydrogène) avec un acide activé ou un chlorure d'acide de formule générale (III) dans laquelle Y représente un groupe OH activé ou un

Schéma 1

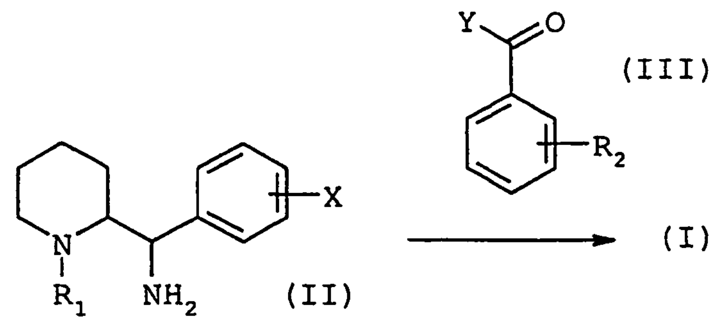

atome de chlore et $R_2$ est tel que défini ci-dessus, en utilisant les méthodes connues de l'homme du métier.

La diamine de formule générale (II) peut être préparée par un procédé illustré par le schéma 2 qui suit.

Schéma 2

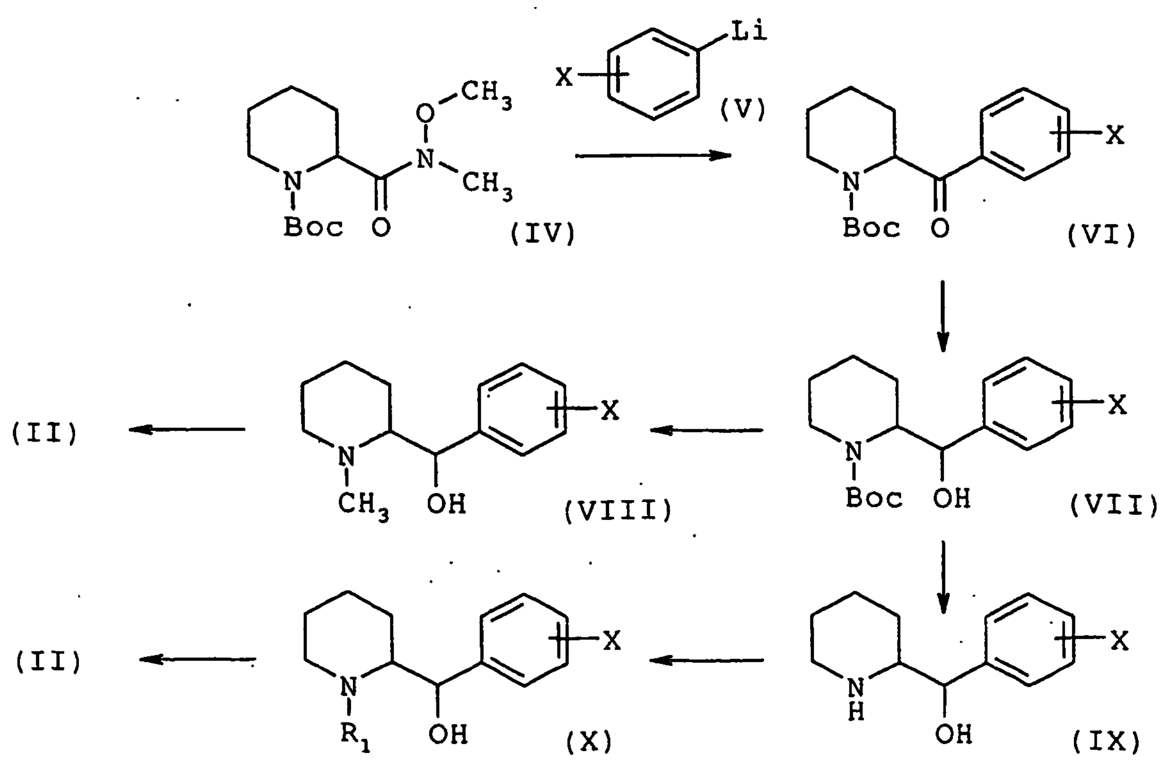

On fait réagir l'amide de Weinreb de formule (IV) avec le dérivé de phényllithium de formule générale (V), dans laquelle X est tel que défini ci-dessus, dans un solvant éthéré tel que l'éther diéthylique, entre -30°C et la température ambiante ; on obtient une cétone de formule générale (VI) que l'on réduit en alcool de configuration thréo de formule générale (VII) par un agent réducteur tel que le K-Selectride® ou le L-Selectride® (tri-sec-butylborohydrure de potassium ou de lithium), dans un solvant éthéré tel que le tétrahydrofurane, entre -78°C et la température ambiante. Le carbamate de formule générale (VII) peut ensuite être réduit en N-méthylaminoalcool thréo de formule générale (VIII) par action d'un hydrure mixte tel que l'hydrure double d'aluminium et de lithium, dans un solvant éthéré tel que le tétrahydrofurane, entre la température ambiante et la température de reflux. On transforme ensuite l'alcool thréo de formule générale

(VIII) en intermédiaire thréo de formule générale (II) où $R_1$ représente un groupe méthyle en deux étapes : on transforme d'abord la fonction alcool en groupe nucléofuge, par exemple un groupe méthanesulfonate, par action du chlorure de méthylsulfonyle, dans un solvant chloré tel que le dichlorométhane, et en présence d'une base telle que la triéthylamine, entre 0°C et la température ambiante, puis on fait réagir le groupe nucléofuge avec de l'ammoniac liquéfié à -50°C, dans un alcool tel que l'éthanol, dans un milieu clos tel qu'un autoclave, entre -50°C et la température ambiante.

On peut également déprotéger le carbamate de formule générale (VII) au moyen d'une base forte telle que la potasse aqueuse, dans un alcool tel que le méthanol pour obtenir l'aminoalcool thréo de formule générale (IX), procéder ensuite à une N-alkylation au moyen d'un dérivé halogéné de formule $R_1Z$, dans laquelle $R_1$ est tel que défini ci-dessus, mais différent d'un atome d'hydrogène, et Z représente un atome d'halogène, en présence d'une base telle que le carbonate de potassium, dans un solvant polaire tel que le *N,N*-diméthylformamide, entre la température ambiante et 100°C. On traite ensuite l'alcool de formule générale (X) ainsi obtenu comme décrit à propos de l'alcool de formule générale (VIII).

Une autre variante de procédé, illustrée par le schéma 3 qui suit, peut être utilisée dans le cas où $R_1$ représente un groupe méthyle et X représente un atome d'hydrogène. On quaternarise la pyridineoxime de formule (XI), par exemple par action du trifluorométhanesulfonate de méthyle, dans un solvant éthéré tel que l'éther diéthylique à température ambiante. On soumet ensuite le sel de pyridinium ainsi obtenu, de formule (XII) à une hydrogénation sous atmosphère d'hydrogène, en présence d'un

Schéma 3

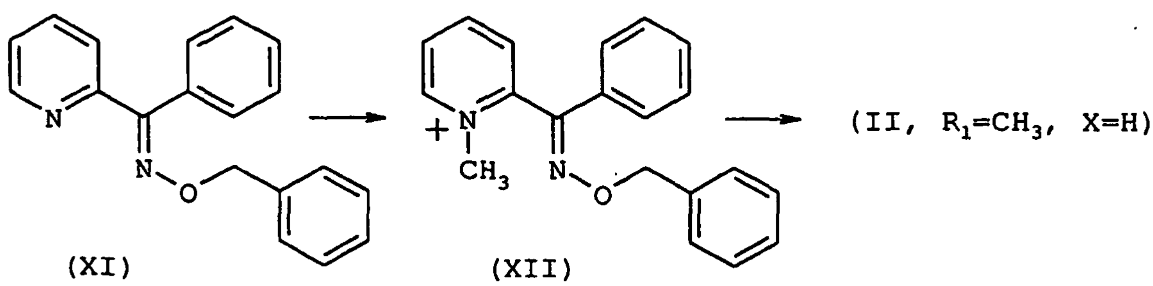


(XI) (XII)

catalyseur tel que l'oxyde de platine, dans un mélange d'alcool et d'acide aqueux tel que l'éthanol et l'acide chlorhydrique 1N. On obtient la diamine de formule générale (II) où $R_1$ représente un groupe méthyle et X représente un atome d'hydrogène sous forme d'un mélange des deux diastéréoisomères thréo/érythro 9/1. On peut la salifier, par exemple avec de l'acide oxalique, puis la purifier par recristallisation de l'oxalate formé dans un mélange d'alcool et d'un solvant éthéré tel que le méthanol et l'éther diéthylique, pour obtenir le diastéréoisomère thréo (1*R*,2*R* ; 1*S*,2*S*) pur.

Les composés de formule générale (II) dans laquelle $R_1$ représente un atome d'hydrogène peuvent être préparés par un procédé illustré par le schéma 4 qui suit.

A partir de l'amine de formule générale (XIII), dans laquelle X est tel que défini ci-dessus, on effectue un couplage avec un acide activé ou un chlorure d'acide, tel que décrit ci-dessus, de formule générale (III), selon des

Schéma 4

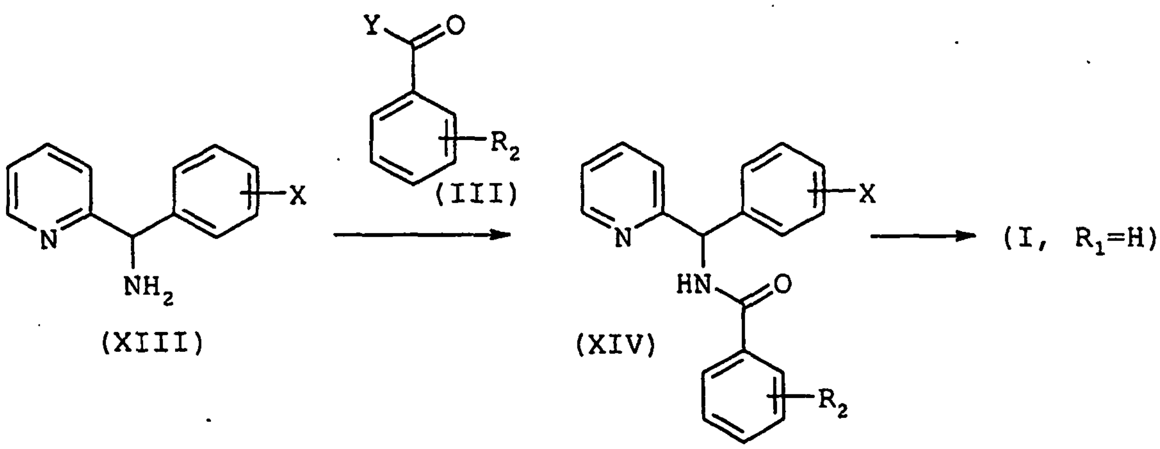

méthodes connues de l'homme du métier, pour obtenir le composé de formule générale (XIV). Finalement on effectue une hydrogénation de ce dernier, par exemple par l'hydrogène en présence d'un catalyseur tel que le platine à 5% sur charbon, dans un solvant acide tel que l'acide acétique glacial, pour obtenir un composé de formule générale (I) dans laquelle $R_1$ représente un atome d'hydrogène.

Une autre méthode consiste à modifier un composé de formule générale (I) dans laquelle $R_1$ représente soit un groupe phénylméthyle éventuellement substitué et à déprotéger l'azote du cycle pipéridine, par exemple par un agent oxydant ou par un acide de Lewis, tel que le tribromure de bore, ou par hydrogénolyse, soit un groupe alcényle, de préférence allyle, et à déprotéger l'azote par un complexe du Pd°, pour obtenir un composé de formule générale (I) dans laquelle $R_1$ représente un atome d'hydrogène.

Par ailleurs les composés chiraux de formule générale (I) correspondant aux énantiomères (1*R*,2*R*) ou (1*S*, 2*S*) du diastéréoisomère thréo peuvent être également obtenus par séparation des composés racémiques par chromatographie liquide à haute performance (CLHP) sur colonne chirale, ou par dédoublement de l'amine racémique de formule générale (II) par utilisation d'un acide chiral tel que l'acide tartrique, l'acide camphorsulfonique, l'acide dibenzoyltartrique ou la N-acétylleucine, par la recristallisation fractionnée et préférentielle d'un sel diastéréoisomérique, dans un solvant de type alcool, ou encore par synthèse énantiosélective selon le schéma 2 avec utilisation d'un amide de Weinreb chiral de formule (IV).

L'amide de Weinreb de formule (IV) racémique ou chiral, ainsi que la cétone de formule générale (VI), peuvent être préparés selon une méthode analogue à celle décrite dans Eur. J. Med. Chem., 35, (2000), 979-988 et J. Med. Chem., 41, (1998), 591-601.,Le composé phényllithié de formule générale (V) où X représente un atome d'hydrogène est disponible dans le commerce. Ses dérivés substitués peuvent être préparés selon une méthode analogue à celle décrite dans Tetrahedron Lett., 57, 33, (1996), 5905-5908. La pyridineoxime de formule générale (XI) peut être préparée selon une méthode analogue à celle décrite dans la demande de brevet EP-0366006. L'amine de formule générale (IX) dans laquelle X représente un atome d'hydrogène peut être préparée en série chirale selon une méthode décrite dans le brevet US-2928835. Enfin, l'amine de formule générale (XIII) peut être préparée selon une méthode analogue à celle décrite dans Chem. Pharm. Bull., 32, 12, (1984), 4893-4906 et Synthesis, (1976), 593-595.

Certains acides et chlorures d'acides de formule générale (III) sont disponibles dans le commerce ou, lorsqu'ils sont nouveaux, ils peuvent être obtenus selon des méthodes analoques à celles décrites dans les brevets EP-0556672, US-3801636, et dans J. Chem. Soc., (1927), 25, Chem. Pharm. Bull., (1992), 1789-1792, Aust. J. Chem., (1984), 1938-1950 et J. O. C., (1980), 527.

Les exemples qui vont suivre illustrent la préparation de quelques composés de l'invention. Les microanalyses élémentaires, les spectres I.R. et R.M.N. et la CLHP sur colonne chirale confirment les structures et les puretés énantiométriques des composés obtenus.

Les numéros indiqués entre parenthèses dans les titres des exemples correspondent à ceux de la 1ère colonne du tableau donné plus loin.

Dans les noms des composés, le tiret "-" fait partie du mot, et le tiret "_" ne sert que pour la coupure en fin de ligne ; il est à supprimer en l'absence de coupure, et ne doit être remplacé ni par un tiret normal ni par un espace.

Exemple 1 (Composé N°65).

Chlorhydrate de 2,3-dichloro-*N*-[(1*S*)-[(2*S*)-1-méthylpipéridin-2-yl]phénylméthyl]benzamide 1:1.

1.1. (2*S*)-2-benzoylpipéridine-1-carboxylate de 1,1-diméthyléthyle.

Dans un ballon de 500 ml, sous atmosphère d'azote, on introduit 11,8 g (43,3 mmoles) de (2*S*)-2-(*N*-méthoxy-*N*-méthylcarbamoyl)pipéridine-1-carboxylate de 1,1-diméthyléthyle dans 100 ml d'éther diéthylique anhydre, on refroidit le milieu à -23°C, on ajoute, goutte à goutte, 21,6 ml (43,2 mmoles) d'une solution 1,8M de phényllithium dans un mélange 70/30 de cyclohexane et d'éther diéthylique et on agite le mélange à température ambiante pendant 3 h. Après hydrolyse avec une solution aqueuse saturée de chlorure de sodium on sépare la phase aqueuse et on l'extrait avec de l'acétate d'éthyle. On sèche la phase organique sur sulfate de sodium, on la filtre, on la concentre sous pression réduite et on purifie le résidu par chromatographie sur colonne de gel de silice en éluant avec un mélange d'acétate d'éthyle et de cyclohexane.
On obtient 4,55 g de produit solide.
Point de fusion : 123-125°C.

$[\alpha]_D^{25} = -25,4°$ (c=2,22 ; $CH_2Cl_2$) ee=97,2%.

1.2. (1*S*)-2-[(2*S*)-hydroxy(phényl)méthyl]pipéridine-1-carboxylate de 1,1-diméthyléthyle.

Dans un ballon de 500 ml, sous atmosphère d'azote, on introduit 4,68 g (16,2 mmoles) de (25)-2-benzoylpipéridine-1-carboxylate de 1,1-diméthyléthyle dans 170 ml de tétrahydrofurane anhydre, on refroidit la solution à -78°C, on ajoute, goutte à goutte , 48,5 ml (48,5 mmoles) d'une solution 1M de L-Selectride® (tri-*sec*-butylborohydrure de lithium) dans le tétrahydrofurane, et on agite le mélange à température ambiante pendant 5 h.
On l'hydrolyse à froid lentement avec 34 ml d'eau et 34 ml d'une solution aqueuse à 35% de peroxyde d'hydrogène, et on laisse le mélange revenir à température ambiante en l'agitant pendant 2 h.
On le dilue avec de l'eau et de l'acétate d'éthyle, , on sépare la phase aqueuse, on l'extrait avec de l'acétate d'éthyle. Après lavage des phases organiques réunies, séchage, séchage sur sulfate de sodium, filtration et évaporation on purifie le résidu par chromatographie sur colonne de gel de silice en éluant avec un mélange d'acétate d'éthyle et de cyclohexane.
On obtient 4,49 g d'une huile jaune pâle.

$[\alpha]_D^{25} = +63,75°$ (c=0,8 ; $CH_2Cl_2$) ee=97,8%.

1.3. (1*S*)-[(2*S*)-(1-méthylpipéridin-2-yl)]phénylméthanol.

Dans un bicol de 200 ml, sous atmosphère d'azote, on introduit 2,96 g (78,1 mmoles) d'hydrure double d'aluminium et de lithium dans 50 ml de tétrahydrofurane anhydre, on chauffe le mélange au reflux, on ajoute 4,49 g (15,4 mmoles) d'une solution de (1*S*)-2-[(2*S*)-hydroxy(phényl)-méthyl]pipéridine-1-carboxylate de 1,1-diméthyléthyle dans 35 ml de tétrahydrofurane et on maintient le mélange au reflux pendant 3,5 h.
On le refroidit, on l'hydrolyse lentement avec une solution 0,1M de tartrate double de potassium et de sodium et on laisse le mélange sous agitation pendant une nuit.
On le filtre et on rinçe le précipité avec du tétrahydrofurane, puis on concentre le filtrat sous pression réduite.
On obtient 2,95 g d'un produit huileux incolore.

1.4. (1*S*)-[(2*S*)-(1-méthylpipéridin-2-yl)]phénylméthanamine.

Dans un ballon de 250 ml, sous atmosphère d'azote, on introduit 2, 95 g (14,4 mmoles) de (1*S*)-[(2*S*)-(1-méthylpipéridin-2-yl)]phénylméthanol et 2 ml (14,4 mmoles) de triéthylamine dans 70 ml de dichlorométhane anhydre, on refroidit le milieu à 0°C, on ajoute 1,1 ml (14,4 mmoles) de chlorure de méthanesulfonyle, on laisse le mélange revenir lentement à température ambiante pendant 2 h et on le concentre sous pression réduite.
Dans un autoclave muni d'une agitation magnétique et refroidi à -50°C on introduit de l'ammoniac liquéfié, on ajoute une solution du méthanesulfonate brut précédemment préparé en solution dans 30 ml d'éthanol absolu, on ferme l'autoclave et on maintient l'agitation pendant 48 h.
On transvase le mélange dans un ballon, on évapore le solvant sous pression réduite, et on isole l'amine sous forme de produit huileux qu'on utilise tel quel dans l'étape suivante.

1.5. Chlorhydrate de 2,3-dichloro-*N*-[(1*S*)-[(2*S*)-1-méthylpipéridin-2-yl]phénylméthyl]benzamide 1:1.

Dans un ballon de 50 ml on introduit 0,5 g (2,6 mmoles) d'acide 2,3-dichlorobenzoïque, 0,5 g (2,6 mmoles) de chlorhydrate de 1-[3-(diméthylamino)propyl]-3-éthylcarbodiimide, 0,35 g (2,6 mmoles) de 1-hydroxybenzotriazole en solution dans 10 ml de dichlorométhane et on agite le mélange à température ambiante pendant 30 min. On ajoute 0,53 g (2,5 mmoles) de
(1*S*)-[(2*S*)-(1-méthylpipéridin-2-yl)]phénylméthanamine en solution dans quelques ml de dichlorométhane et on poursuit l'agitation pendant 5 h.
On traite le mélange avec de l'eau, on l'extrait plusieurs fois avec du dichlorométhane. Après lavage des phases organiques avec de l'eau puis avec une solution aqueuse de soude 1N, séchage sur sulfate de magnésium, filtration et évaporation du solvant sous pression réduite, on purifie le résidu par chromatographie sur colonne de gel de silice en éluant avec un mélange de dichlorométhane et de méthanol.
On obtient 0,52 g de produit huileux qu'on isole sous forme de chlorhydrate à partir d'une solution 0,1N d'acide chlorhydrique dans le propan-2-ol.
On isole finalement 0,5 g de chlorhydrate sous forme de solide blanc.
Point de fusion : 124-126°C.

$[\alpha]_D^{25} = +66,3°$ (C=0,58 ; $CH_3OH$).

<u>Exemple 2</u> (Composé N°55).

Chlorhydrate de 4-amino-3,5-dichloro-*N*-[(1*R*)-[(2*R*)-1-méthylpipéridin-2-yl]phénylméthyl]benzamide 1:1.

2.1. Trifluorométhanesulfonate de 2-(benzyloxyiminophénylméthyl)-1-méthylpyridinium.

A une suspension de 35 g (120 mmoles) de phényl(pyridin-2-yl)méthanone O-benzyloxime dans 200 ml d'éther diéthylique on ajoute, goutte à goutte et à 0°C, 17,4 ml (120 mmoles) de trifluorométhanesulfonate de méthyle et on agite le mélange à température ambiante pendant 3 h.
On recueille le précipité formé par filtration et on le sèche sous pression réduite.
On obtient 49 g de produit qu'on utilise tel quel dans l'étape suivante.

2.2. Ethanedioate de thréo-(1-méthylpipéridin-2-yl)-phénylméthanamine 2:1.

Dans une fiole de Parr on place 14,8 g (31,89 mmoles) de trifluorométhane sulfonate de 2-(benzyloxyiminophénylméthyl)-1-méthylpyridinium et 0,74 g d'oxyde de platine dans 50 ml d'éthanol et 50 ml d'acide chlorhydrique 1N et on effectue une hydrogénation pendant 5 h.
On évapore l'éthanol sous pression réduite, on extrait le résidu avec du dichlorométhane, on sépare la phase aqueuse, on y ajoute une solution d'ammoniaque et on l'extrait avec du dichlorométhane. Après lavage des phases organiques réunies, séchage sur sulfate de sodium, filtration et évaporation du solvant sous pression réduite, on obtient 6,7 g de produit huileux comprenant 10% de diastéréoisomère érythro.
On prépare l'éthanedioate en dissolvant ces 6,7 g de base dans le méthanol, par action de deux équivalents d'acide éthanedioïque dissous dans le minimum de méthanol.
On purifie le sel obtenu par recristallisation dans un mélange de méthanol et d'éther diéthylique.
On isole finalement 4,7 g d'éthanedioate du diastéréoisomère thréo pur.
Point de fusion : 156-159°C.

2.3. (1*R*)-[(2*R*)-(1-méthylpipéridin-2-yl)]phénylméthanamine.

Dans un ballon de 4 1 on introduit 80 g (390 mmoles) de thréo-(1-méthylpipéridin-2-yl)phénylméthanamine en solution dans 300 ml de méthanol et 68 g (390 mmoles) de N-acétyl-D-leucine en solution dans 450 ml de méthanol. On concentre la solution sous pression réduite et on recristallise le résidu dans 1100 ml de propan-2-ol. On obtient 72 g de sels de (1*R*)-[(2*R*)-(1-méthylpipéridin-2-yl)]phénylméthanamine.
On réitère trois fois la recristallisation et on obtient finalement 15 g de sel de (1*R*)-[(2*R*)-(1-méthylpipéridin-2-yl)]phénylméthanamine.
Point de fusion : 171,5°C.

$[\alpha]_D^{25} = -11°$ (c=1 ; $CH_3OH$) ee>99%.

2.4. Chlorhydrate de 4-amino-3,5-dichloro-*N*-[(1*R*)-[(2*R*)-1-méthylpipéridin-2-yl]phénylméthyl]-3,5-dichloro benzamide 1:1.

En utilisant le mode opératoire décrit au point 1.6 ci-dessus, à partir de 2,18 g (11,65 mmoles) d'acide 4-amino-3,5-dichlorobenzoïque, de 2,23 g (10,6 mmoles) de chlorhydrate de 1-[3-(diméthylamino)propyl]-3-éthylcarbodiimide, de 1,41 g (10,6 mmoles) de 1-hydroxybenzotriazole et de 2,16 g (10,6 mmoles) de (1*R*)-[(2*R*)-méthylpipéridin-2-yl)]phénylméthanamine, on obtient 3,92 g de produit sous forme de base.
On en prépare le chlorhydrate à partir d'une solution O,1N d'acide chlorhydrique dans le propan-2-ol.
On isole finalement 3,94 g de chlorhydrate sous forme de solide blanc.
Point de fusion : 250-260°C.

$[\alpha]_D^{25} = +24,5°$ (c=0,9 ; $CH_3OH$).

<u>Exemple 3</u> (Composé N°59).

Chlorhydrate de 3,5-dichloro-*N*-[(1-méthylpipéridin-2-yl)-phénylméthyl]-4-(pyrrolidin-1-yl)benzamide 1:1.

3.1. Acide 3,5-dichloro-4-fluorobenzoïque.

Dans un autoclave on introduit 5 g (21,46 mmoles) de 3,5-dichloro-4-fluoro[(trifluorométhyl)benzène] en solution dans 10 ml d'acide sulfurique concentré et on chauffe la solution à 120°C pendant une nuit.
Après refroidissement on reprend le mélange avec de l'eau, on recueille le précipité formé par filtration et on le sèche sous pression réduite.
On obtient quantitativement l'acide qu'on utilise tel quel dans l'étape suivante.

3.2. Acide 3,5-dichloro-4-(pyrrolidin-1-yl)benzoïque.

Dans un ballon de 100 ml on introduit 1 g (4,8 mmoles) d'acide 3,5-dichloro-4-fluorobenzoïque, 1,56 g (4,8 mmoles) de carbonate de césium et 1 ml (12 mmoles) de pyrrolidine en solution dans 5 ml de diméthylsulfoxyde et on chauffe le mélange à 125 pendant une nuit.
Après refroidissement on l'hydrolyse avec de l'acide chlorhydrique concentré, on recueille le précipité formé par filtration et on le sèche sous pression réduite.
On obtient 0,65 g d'acide.

3.3. Chlorhydrate de 3,5-dichloro-N-[(1-méthylpipéridin-2-yl)phénylméthyl]-4-(pyrrolidin-1-yl)benzamide 1:1.

En utilisant le mode opératoire décrit au point 1.6 ci-dessus, à partir de 0,5 g (2 mmoles) d'acide 3,5-dichloro-4-(pyrrolidin-1-yl)benzoïque, de 0,35 g (1,82 mmoles) de chlorhydrate de 1-[3-(diméthylamino)propyl]-3-éthylcarbodiimide, de 0,25 g (1,82 mmoles) de 1-hydroxybenzotriazole et de 0,37 g (1,82 mmoles) de thréo-(1-méthylpipéridin-2-yl)]phénylméthanamine, on obtient 0,1 g de produit sous forme de base.
On en prépare le chlorhydrate à partir d'une solution 0,1N d'acide chlorhydrique dans le propan-2-ol.
On isole finalement 0,85 g de chlorhydrate sous forme de solide blanc.
Point de fusion : 157-159°C.

<u>Exemple 4</u> (Composé N°76).

2,3-dichloro-*N*-[(*S*)-phényl[(2*S*)-pipéridin-2-yl]méthyl]benzamide.

4.1. (*S*)-phényl[(2*S*)-pipéridin-2-yl]méthanol.

Dans un ballon de 250 ml on place une solution de 2,0 g (6,9 mmoles) de (1*S*)-2-[(2*S*)-hydroxy(phényl)méthyl] pipéridine-1-carboxylate de 1,1-diméthyléthyle (obtenu selon le mode opératoire décrit dans l'étape 1.2 de l'exemple 1) dans 40 ml de méthanol, on ajoute une solution aqueuse de potasse préparée à partir de 2 g de potasse en pastilles et 20 ml d'eau, et on chauffe le mélange au reflux pendant 2 h.
On le refroidit, on évapore le solvant sous pression réduite, on ajoute de l'eau et on extrait le mélange plusieurs fois avec du dichlorométhane. Après lavage des phases organiques réunies, séchage sur sulfate de magnésium, filtration et évaporation du solvant sous pression réduite on obtient 1 g de solide blanc.

Point de fusion : 148-150°C.

$[\alpha]_D^{25} = +38,4°$ (c=0, 98 ; $CHCl_3$).

4.2. (*S*)-[(2*S*)-1-allylpipéridin-2-yl](phényl)méthanol.

Dans un ballon de 500 ml muni d'une agitation magnétique et d'une circulation d'argon on introduit 2,6 g (13,58 mmoles) de (*S*)-phényl[(2*S*)-pipéridin-2-yl]méthanol et 100 ml d'acétonitrile. A la suspension obtenue on ajoute ensuite 2,8 g de carbonate de potassium et 1,4 ml (1,2 éq.)de bromure d'allyle, et on maintient l'agitation à 25°C pendant 6 h.
On ajoute 100 ml d'eau et 100 ml d'acétate d'éthyle, on sépare la phase aqueuse, on l'extrait trois fois avec 50 ml d'acétate d'éthyle, on lave les phases organiques réunies avec 100 ml d'eau puis 100 ml d'une solution aqueuse saturée de chlorure de sodium, on sèche sur sulfate de sodium, on filtre et on évapore le solvant sous pression réduite.
On obtient 3 g d'huile jaune que l'on purifie par chromatographie sur colonne de gel de silice (120 g, gradient d'élution de 2% à 10% de méthanol dans le dichlorométhane en 30 min).
On isole 2,7 g de produit sous forme d'huile jaune.

4.3. (*S*)-[(2*S*)-1-allylpipéridin-2-yl](phényl)méthanamine.

Dans un ballon de 250 ml, sous atmosphère d'azote, on introduit 2,7 g (11,67 mmoles) de (*S*)-[(2*S*)-1-allylpipéridin-2-yl](phényl)méthanol et 1,62 ml de triéthylamine dans 80 ml de dichlorométhane anhydre, on refroidit le milieu à 0°C, on ajoute 0,9 ml de chlorure de méthylsulfonyle, on laisse le mélange revenir lentement à température ambiante pendant 2 h et on le concentre sous pression réduite.
Dans un autoclave muni d'une agitation magnétique et refroidi à -50°C on introduit de l'ammoniac liquéfié, on ajoute une solution de méthanesulfonate brut précédemment préparé en solution dans 30 ml d'éthanol absolu, on ferme l'autoclave et on maintient l'agitation pendant 48 h.
On transvase le mélange dans un ballon, on le concentre sous pression réduite et on isole 1,5 g d'amine sous forme de produit huileux qu'on utilise tel quel dans l'étape suivante.

4.4. *N*-[(*S*)-[(2*S*)-1-allylpipéridin-2-yl](phényl)méthyl]-2,3-dichlorobenzamide.

Dans un ballon de 10 ml on introduit successivement, dans 5 ml de dichlorométhane, 0,13 g (0,68 mmole) d'acide 2,3-dichlorobenzoïque, 0,13 g (0,68 mmole) de chlorhydrate de 1-[3-(diméthylamino)propyl]-3-éthylcarbodiimide et 0,085 g de diméthylaminopyridine, et on agite le mélange à température ambiante pendant 30 min.
On ajoute 0,18 g de (*S*)-[(2*S*)-1-allylpipéridin-2-yl]-(phényl)méthanamine en solution dans quelques ml de dichlorométhane et on poursuit l'agitation pendant 24 h.
On ajoute 5 ml d'eau, on filtre sur cartouche Whatman® (PTFE) et on purifie directement sur cartouche de 10 g de silice en éluant avec un mélange 98/2 à 90/10 de dichloro méthane et de méthanol.
On isole 0,18 g de base sous forme d'huile incolore.

4.5. 2,3-dichloro-*N*-[(*S*)-phényl[(2*S*)-pipéridin-2-yl] méthyl]benzamide .

Dans un ballon de 10 ml muni d'uni d'une agitation magnétique on introduit, sous atmosphère d'argon, 0,21 g (3 éq.) d'acide 1,3-diméthylbarbiturique en solution dans 3 ml de dichlorométhane anhydre, on ajoute 0,005 g (0,01 éq.) de tétrakis(triphénylphosphine)palladium (0) et on chauffe le mélange à 30°C.

On ajoute une solution de 0,18 g (0,3 mmole) de *N*-[(*S*)-[(2*S*)-1-allylpipéridin-2-yl](phényl)méthyl]-2,3-dichlorobenzamide dans 1 ml de dichlorométhane et on laisse le mélange sous agitation pendant 24 h.
On ajoute 3 ml d'une solution aqueuse saturée d'hydrogénosulfate de sodium, on on filtre sur cartouche Whatman® (PTFE) et on purifie directement sur cartouche de 10 g de silice en éluant avec du dichlorométhane contenant 0,4% d'ammonique à 33%.
On isole 0,1 g de base que l'on salifie par une solution O,1N d'acide chlorhydrique dans le propan-2-ol.
On obtient 0,076 g de chlorhydrate qu'on purifie en phase inverse sur colonne XTerra® MS C18 (pH 10).
On isole finalement 0,037 g de base sous forme de cristaux blancs.
Point de fusion : 156-158°C.
Le tableau de la page suivante illustre les structures chimiques et les propriétés physiques de quelques composés de l'invention.
Dans la colonne "$R_2$" de ce tableau, "piperid" désigne un groupe pipéridin-1-yle, "pyrrolid" désigne un groupe pyrrolidin-1-yle et "morphol" désigne un groupe morpholin-4-yle.

Dans la colonne "Sel", "-" désigne un composé à l'état de base et "HCl" désigne un chlorhydrate ; le rapport molaire acide:base est indiqué en regard.

Les pouvoirs rotatoires des composés optiquement purs sont les suivants

| N° | stéréochimie | $[\alpha]_D^{20}$ (°, $CH_3OH$) | |
|---|---|---|---|
| 41 | thréo (1*S*,2*S*) | -73,3 | c=0,225 |
| 55 | thréo (1*R*,2*R*) | +24,5 | c=0, 9 |
| 64 | thréo (1*S*,2*S*) | +13,2 | c=0, 84 |
| 65 | thréo (1*S*,2*S*) | +66, 3 | c=0, 58 |
| 71 | thréo (1*S*,2*S*) | +73,9 | c=0, 89 |
| 72 | thréo (1*R*,2*R*) | -97,0 | c=1 |
| 74 | thréo (1*R*,2*R*) | -104,4 | c=1 |

Tableau

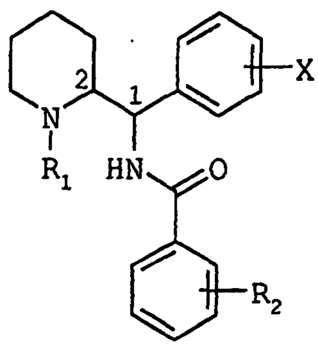

(I)

| N° | stéréochimie | $R_1$ | X | $R_2$ | Sel | | F (°C) |
|---|---|---|---|---|---|---|---|
| 1 | thréo (1*R*,2*R*;1*S*,2*S*) | $CH_3$ | H | 3-$OCH_3$, 4-Cl | - | | 159-161 |
| 2 | thréo (1*R*,2*R*;1*S*,2*S*) | $CH_3$ | H | 3-I, 4-Cl | - | | 102-104 |
| 3 | thréo (1*R*,2*R*;1*S*,2*S*) | $CH_3$ | H | 4-Cl | - | | 149, 5-150, 5 |
| 4 | thréo (1*R*,2*R*;1*S*,2*S*) | $CH_3$ | H | 3,5-$Cl_2$ | HCl | 1:1 | 152-154 |
| 5 | thréo (1*R*,2*R*;1*S*,2*S*) | $CH_3$ | H | 4-$N(CH_3)_2$ | - | | 128-130 |
| 6 | thréo (1*R*,2*R*;1*S*,2*S*) | $CH_3$ | H | 3,4-$Cl_2$ | - | | 50-52 |
| 7 | thréo (1*R*,2*R*;1*S*,2*S*) | $CH_3$ | H | 3,4-$(OCH_3)_2$ | HCl | 1:1 | 68-70 |
| 8 | thréo (1*R*,2*R*;1*S*,2*S*) | $CH_3$ | H | 3,4-$F_2$ | HCl | 1:1 | 62-64 |
| 9 | thréo (1*R*,2*R*;1*S*,2*S*) | $CH_3$ | H | 3-F | HCl | 1:1 | 36-38 |
| 10 | thréo (1*R*, 2*R*; 1*S*, 2*S*) | $CH_3$ | H | 3-Br | HCl | 1:1 | 116-118 |
| 11 | thréo (1*R*, 2*R*; 1*S*, 2*S*) | $CH_3$ | H | 3, 5-$Cl_2$, 4-OH | - | | 266-268 |
| 12 | thréo (1*R*, 2*R*; 1*S*, 2*S*) | $CH_3$ | H | 3-$N(CH_3)_2$ | HCl | 1:1 | 87-88 |
| 13 | thréo (1*R*, 2*R*; 1*S*, 2*S*) | $CH_3$ | H | 3, 5-$Cl_2$, 4-$NH_2$ | - | | 170-171 |
| 14 | thréo (1*R*, 2*R*; 1*S*, 2*S*) | $CH_3$ | H | 3-Br, 4-$OCH_3$ | HCl | 1:1 | 136-137 |
| 15 | thréo (1*R*, 2*R*; 1*S*, 2*S*) | $CH_3$ | H | 2,4,6-$Cl_3$ | - | | 97-104 |
| 16 | thréo (1*R*, 2*R*; 1*S*, 2*S*) | $CH_3$ | H | 2,3-$Cl_2$ | - | | 107-114 |
| 17 | thréo (1*R*, 2*R*; 1*S*, 2*S*) | $CH_3$ | H | 2-Cl | - | | 126-130 |
| 18 | thréo (1*R*, 2*R*; 1*S*, 2*S*) | $CH_3$ | H | 2,4-$Cl_2$ | - | | 138-142 |
| 19 | thréo (1*R*,2*R*;1*S*,2*S*) | $CH_3$ | H | 2-Cl, 4-Br | - | | 143-145 |
| 20 | thréo (1*R*,2*R*;1*S*,2*S*) | $CH_3$ | H | 2,5-$Cl_2$ | - | | 133-134 |
| 21 | thréo (1*R*, 2*R*, 1*S*, 2*S*) | $CH_3$ | H | 2,6-$Cl_2$ | - | | 138-143 |
| 22 | thréo (1*R*, 2*R*; 1*S*, 2*S*) | $CH_3$ | H | 2,3,5-$Cl_3$ | - | | 156-159 |

(suite)

| | | | | | | | |
|---|---|---|---|---|---|---|---|
| 23 | thréo (1*R*, 2*R*; 1*S*, 2*S*) | $CH_3$ | H | 2-$NH_2$, 3,5-$Cl_2$ | HCl | 1:1 | 186-188 |
| 24 | thréo (1*R*, 2*R*; 1*S*, 2*S*) | $CH_3$ | H | 2-Cl, 5-$NH_2$ | HCl | 1:1 | 266-268 |
| 25 | thréo (1*R*, 2*R*; 1*S*, 2*S*) | $CH_3$ | H | 3-Cl, 4-$NH_2$ | HCl | 1:1 | 164-166 |
| 26 | thréo (1*R*, 2*R*; 1*S*, 2*S*) | $CH_3$ | H | 3-$NH_2$, 4-Cl | HCl | 1:1 | 230-232 |
| 27 | thréo (1*R*, 2*R*; 1*S*, 2*S*) | $CH_3$ | H | 2-Cl, 4-$NH_2$ | HCl | 1:1 | 254-256 |
| 28 | thréo (1*R*, 2*R*; 1*S*, 2*S*) | $CH_3$ | H | 2-$NH_2$, 4-Cl | HCl | 1:1 | 236-238 |
| 29 | thréo (1*R*, 2*R*; 1*S*, 2*S*) | $CH_3$ | H | 2-Cl, 3-$NH_2$ | HCl | 1:1 | 195-200 |
| 30 | thréo (1*R*, 2*R*; 1*S*, 2*S*) | $CH_3$ | H | 6-$NH_2$, 2, 5-$Cl_2$ | HCl | 1: 1 | 267-268 |
| 31 | thréo (1*R*, 2*R*; 1*S*, 2*S*) | $CH_3$ | H | 3,5-$Cl_2$, 4-O $(CH_2)_2$piperid | HCl | 2:1 | 44-46 |
| 32 | thréo (1*R*, 2*R*; 1*S*, 2*S*) | $CH_3$ | H | 3,5-$Cl_2$, 4-O$(CH_2)_3$N $(CH_3)_2$ | HCl | 2:1 | 39-41 |
| 33 | thréo (1*R*, 2*R*; 1*S*, 2*S*) | $CH_3$ | H | 3, 5-$Cl_2$, 4-O$(CH_2)_2$N $(CH_3)_2$ | HCl | 2: 1 | 130-132 |
| 34 | thréo (1*R*, 2*R*; 1*S*, 2*S*) | $CH_3$ | H | 3, 5-$Cl_2$, 4-O $(CH_2)_2$pyrrolid | HCl | 2:1 | 78-80 |
| 35 | thréo (1*R*, 2*R*; 1*S*, 2*S*) | $CH_3$ | H | 3, 5-$Cl_2$, 4-O $(CH_2)_2$morphol | HCl | 2:1 | 166-168 |
| 36 | thréo (2*R*, 2*R*; 1*S*, 2*S*) | $CH_3$ | H | 2-Cl, 6-F | HCl | 1:1 | 266-268 |
| 37 | thréo (1*R*, 2*R*; 1*S*, 2*S*) | $CH_3$ | H | 3, 5-$Cl_2$, 4-$N(CH_3)_2$ | HCl | 1:1 | 157-159 |
| 38 | thréo (1*R*, 2*R*; 1*S*, 2*S*) | $CH_3$ | H | 2-Cl, 5-I | HCl | 1:1 | 281-285 |
| 39 | thréo (1*R*,2*R*;1*S*,2*S*) | $CH_3$ | H | 3,5-$Cl_2$, 4-$NHCH_2CH_3$ | HCl | 1:1 | 175-180 |
| 40 | thréo (1*R*,2*R*;1*S*,2*S*) | $CH_3$ | H | 3-Cl, 4-I | HCl | 1:1 | 98-99 |
| 41 | thréo (1*S*,2*S*) | $CH_3$ | H | 3,5-$Cl_2$, 4-$NH_2$ | - | | 176-177 |
| 42 | thréo (1*R*,2*R*,1*S*,2*S*) | $CH_3$ | H | 2-I, 4-Cl | - | | 213-214 |
| 43 | thréo (1*R*,2*R*;1*S*,2*S*) | $CH_3$ | H | 3-Cl, 4-OH | HCl | 1:1 | 194-195 |
| 44 | thréo (1*R*,2*R*;1*S*,2*S*) | $CH_3$ | H | 3-Cl, 4-piperid | HCl | 1:1 | 270-272 |
| 45 | thréo (1*R*,2*R*;1*S*,2*S*) | $CH_3$ | H | 2-$OCH_3$, 3,5-$Cl_2$ | - | | 97-98 |
| 46 | thréo (1*R*,2*R*;1*S*,2*S*) | $CH_3$ | H | 2-Cl, 3,4-$(OCH_3)_2$ | - | | 229-230 |
| 47 | thréo (1*R*,2*R*;1*S*,2*S*) | $CH_3$ | H | 2-Br, 4-F | - | | 124-125 |
| 48 | thréo (1*R*, 2*R*; 1*S*, 2*S*) | $CH_3$ | H | 3-Cl, 4-pyrrolid | HCl | 1:1 | 154-156 |
| 49 | thréo (1*R*, 2*R*; 1*S*, 2*S*) | $CH_3$ | H | 2-Br, 5-Cl | - | | 156-157 |
| 50 | thréo (1*R*, 2*R*; 1*S*, 2*S*) | $CH_3$ | H | 2-Br | - | | 202-203 |
| 51 | thréo (1*R*, 2*R*, 1*S*, 2*S*) | $CH_3$ | H | 2-Br, 4,5-$(OCH_3)_2$ | - | | 218-219 |
| 52 | thréo (1*R*, 2*R*; 1*S*, 2*S*) | $CH_3$ | H | 2-Cl, 3,6-$F_2$ | - | | 52-53 |
| 53 | thréo (1*R*, 2*R;* 1*S*, 2*S*) | $CH_3$ | H | 3-Cl, 4-morphol | HCl | 1:1 | 158-162 |
| 54 | thréo (1*R*, 2*R*; 1*S*, 2*S*) | $CH_3$ | H | 3,5-Cl, 4-piperid | HCl | 1:1 | 154-163 |
| 55 | thréo (1*R*, 2*R*) | $CH_3$ | H | 3,5-$Cl_2$, 4-$NH_2$ | HCl | 1:1 | 250-260 |
| 56 | thréo (1*R*, 2*R*; 1*S*, 2*S*) | $CH_3$ | H | 2-I, 3-Cl | HCl | 1:1 | 253-255 |
| 57 | thréo (1*R*, 2*R*; 1*S*, 2*S*) | $CH_3$ | H | 2-Cl, 3-I | HCl | 1:1 | 297-298 |
| 58 | thréo (1*R*, 2*R*; 1*S*, 2*S*) | $CH_3$ | H | 3,5-$Cl_2$, 4-$NHC_6H_5$ | HCl | 1:1 | 236-240 |
| 59 | thréo (1*R*, 2*R*; 1*S*, 2*S*) | $CH_3$ | H | 3,5-$Cl_2$, 4-pyrrolid | HCl | 1:1 | 157-159 |
| 60 | thréo (1*R*, 2*R*; 1*S*, 2*S*) | $CH_3$ | H | 2-Cl, 6-F | HCl | 1:1 | 271-272 |
| 61 | thréo (1*R*, 2*R*; 1*S*, 2*S)* | $CH_3$ | H | 2-Cl, 4,5- $(OCH_3)_2$ | - | | 242-243 |
| 62 | thréo (1*R*, 2*R*; 1*S*, 2*S*) | $CH_3$ | H | 2-Cl, 4-F | HCl | 1:1 | 365-366 |
| 63 | thréo (1*R*, 2*R*; 1*S*, 2*S*) | $CH_3$ | H | 2-Br, 5-$OCH_3$ | - | | 213-214 |
| 64 | thréo (1*S*, 2*S*) | $CH_3$ | H | 3,5-$Cl_2$, 4-$N(CH_3)_2$ | HCl | 1:1 | 158-168 |
| 65 | thréo (1*S*, 2*S*) | $CH_3$ | H | 2,3-$Cl_2$ | HCl | 1:1 | 124-126 |
| 66 | thréo (1*R*, 2*R*; 1*S*, 2*S)* | $CH_3$ | H | 3,5-$(OCH_3)_2$, 4-$OCH_2C_6H_5$ | HCl | 1:1 | 274-275 |
| 67 | thréo (1*R*, 2*R;* 1*S*, 2*S*) | $CH_3$ | H | 3, 5-$Cl_2$, 4-$OCH_2C_6H_5$ | HCl | 1 : 1 | 165-175 |

(suite)

| | | | | | | | |
|---|---|---|---|---|---|---|---|
| 68 | thréo (1*R*, 2*R*; *1S*, 2*S)* | $CH_3$ | H | 3,5-$Cl_2$, 4-$NHCH_2C_6H_5$ | HCl | 1:1 | 165-175 |
| 69 | thréo (1*R*, 2*R*; 1*S*, 2*S*) | $CH_3$ | H | 2-Cl, 3-F | HCl | 1:1 | 115-116 |
| 70 | thréo (1*R*, 2*R*; 1*S*, 2*S*) | $CH_3$ | H | 2-Cl, 5-F | HCl | 1:1 | 212-215 |
| 71 | thréo (1*S*, 2*S*) | $CH_3$ | H | 2-Cl, 4-F | HCl | 1:1 | 123-125 |
| 72 | thréo (1*R*, 2*R*) | $CH_3$ | H | 2, 5-$Cl_2$, 6-$NH_2$ | HCl | 1:1 | 66-67 |
| 73 | thréo (1*R*, 2*R;* 1*S*, 2*S*) | $CH_3$ | H | 2-Cl, 5-$OCH_3$, 6-$NH_2$ | HCl | 1:1 | 258-259 |
| 74 | thréo (1*R*, 2*R*) | $CH_3$ | H | 2-Cl, 5-$OCH_3$, 6-$NH_2$ | HCl | 1:1 | 138-139 |
| 75 | thréo (1*R*, 2*R*; 1*S*, 2*S*) | $CH_3$ | H | 2, 3-$Cl_2$, 6-$NH_2$ | HCl | 1:1 | 230-236 |
| 76 | thréo (1*S*, 2*S*) | H | H | 2,3-$Cl_2$ | - | - | 156-158 |

Les composés de l'invention ont été soumis à une série d'essais pharmacologiques qui ont mis en évidence leur intérêt comme substances à activités thérapeutiques.

Etude du transport de la glycine dans les cellules SK-N-MC exprimant le transporteur humain glyt1 natif.

La capture de [$^{14}$C]glycine est étudiée dans les cellules SK-N-MC (cellules neuro-épithéliales humaines) exprimant le transporteur humain glyt1 natif par la mesure de la radioactivité incorporée en présence ou en absence du composé à tester. Les cellules sont cultivées en monocouche pendant 48 h dans des plaques prétraitées à la fibronectine à 0,02%. Le jour de l'expérience, le milieu de culture est éliminé et les cellules sont lavées par un tampon Krebs-HEPES (acide [4-(2-hydroxyéthyl)pipérazine-1-éthanesulfonique) à pH 7,4. Après 10 min de préincubation à 37˚C en présence soit de tampon (lot témoin), soit de composé à tester a différentes concentrations ou de 10 mM de glycine (détermination de la capture non spécifique), 10 μM de [$^{14}$C]glycine (activité spécifique 112 mCi/mmole) sont ensuite ajoutés. L'incubation se poursuit pendant 10 min à 37˚C, et la réaction est arrêtée par 2 lavages avec un tampon Krebs-HEPES à pH 7,4. La radioactivité incorporée par les cellules est alors estimée après ajout de 100 μl de scintillant liquide et agitation pendant 1 h. Le comptage est réalisé sur compteur Microbeta Tri-lux™. L'efficacité du composé est déterminée par la $CI_{50}$, concentration du composé qui diminue de 50% la capture spécifique de glycine, définie par la différence de radioactivité incorporée par le lot témoin et le lot qui a reçu la glycine à 10 mM.

Les composés de l'invention, dans ce test, ont une $CI_{50}$ de l'ordre de 0,001 à 10.μM.

Etude *ex vivo* de l'activité inhibitrice d'un composé sur la capture de la [$^{14}$C]glycine dans l'homogénat cortical de souris.

Des doses croissantes du composé à étudier sont administrées par voie orale (préparation par trituration de la molécule à tester dans un mortier dans une solution de Tween/Methocel™ à 0,5% dans de l'eau distillée) ou intrapéritonéale (dissolution de la molécule à tester dans du sérum physiologique ou prépation par trituration dans un mortier dans une solution de Tween/methocel à 0,5% dans de l'eau, selon la solubilité de la molécule) sur des souris mâles OF1 Iffa Crédo de 20 à 25 g le jour de l'expérience. Le groupe témoin est traité par le véhicule. Les doses en mg/kg, la voie d'administration et le temps de traitement sont déterminés en fonction de la molécule à étudier.

Après euthanasie par décapitation des animaux à un temps donné après l'administration, le cortex de chaque animal est rapidement prélevé sur glace, pesé et conservé à 4˚C ou congelé à -80˚C (dans les deux cas les échantillons sont conservés 1 jour maximum). Chaque échantillon est homogénéisé dans un tampon Krebs-HEPES à pH 7,4 à raison de 10 ml/g de tissu. 20 μl de chaque homogénat sont incubés pendant 10 min à température ambiante en présence de 10 mM de L-alanine et de tampon. La capture non spécifique est déterminée par addition de 10 mM de glycine au groupe témoin. La réaction est arrêtée par filtration sous vide et la radioactivité retenue est estimée par scintillation solide par comptage sur compteur Microbeta Tri-lux™.

Un inhibiteur de la capture de la [$^{14}$C]glycine diminuera la quantité de radioligand incorporée dans chaque homogénat. L'activité du composé est évaluée par sa $DE_{50}$, dose qui inhibe 50% de la capture de la [$^{14}$C]glycine par rapport au groupe témoin.

Les composés de l'invention les plus puissants, dans ce test, ont une $DE_{50}$ de 0,1 à 5 mg/kg par voie intrapéritonéale ou par voie orale.

Etude du transport de la glycine dans l'homogénat de moelle épinière de souris.

La capture de [$^{14}$C]glycine par le transporteur glyt2 est étudiée dans l'homogénat de moelle épinière de souris par la mesure de radioactivité incorporée en présence ou en absence de composé à étudier.

Après euthanasie des animaux (souris mâles OF1 Iffa Crédo pesant 20 à 25 g le jour de l'expérience), la moelle épinière de chaque animal est rapidement prélevée, pesée et conservée sur glace. Les échantillons sont homogénéisés dans un tampon Krebs-HEPES (acide [4-(2-hydroxyéthyl)pipérazine-1-éthanesulfonique), pH 7,4, à raison de 25 ml/g de tissu.
50 µl d'homogénat sont pré-incubés pendant 10 min à 25°C en présence de tampon Krebs-HEPES , pH 7,4 et de composé à étudier à différentes concentrations, ou de 10 mM de glycine pour déterminer la capture non spécifique. La [$^{14}$C]glycine (activité spécifique = 112mCi/mmole) est ensuite ajoutée pendant 10 min à 25°C à la concentration finale de 10 µM. La réaction est arrêtée par filtration sous vide et la radioactivité est estimée par scintillation solide par comptage sur un compteur Microbeta Tri-lux™. L'efficacité du composé est déterminée par la concentration $CI_{50}$ capable de diminuer de 50% la capture spécifique de glycine, définie par la différence de radioactivité incorporée par le lot témoin et le lot qui a reçu la glycine 10 mM.
Les composés de l'inventiondans ce test, ont une $CI_{50}$ de l'ordre de 0,001 à 10 µM.

Etude *ex vivo* de l'activité inhibitrice d'un composé sur la capture de la [$^{14}$C]glycine dans l'homogénat spinal de souris.

Des doses croissantes du composé à étudier sont aministrées par voie orale (préparation par trituration du composé à tester dans un mortier, dans une solution de Tween/Methocel™ à 0,5% dans de l'eau distillée) ou intrapéritonéale (composé à tester dissous dans du sérum physiologique, ou trituré dans un mortier, dans une solution de Tween/Methocel™ à 0,5% dans de l'eau distillée) à des souris mâles OF1 Iffa Crédo de 20 à 25 g le jour de l'expérience. Le groupe témoin est traité par le véhicule. Les doses en mg/kg, la voie d'administration, le temps de traitement ainsi que le temps d'euthanasie sont déterminés en fonction du composé à étudier.
Après euthanasie par décapitation des animaux à un temps donné après l'administration, les moelles sont prélevées rapidement, pesées et introduites dans des fioles à scintillation en verre, conservées dans de la glace pilée
ou congelées à -80°C (dans les deux cas les échantillons sont conservés 1 jour maximum). Chaque échantillon est homogénéisé dans un tampon Krebs-HEPES à pH 7,4, à raison de 25 ml/g de tissu. 50 µl de chaque homogénat sont incubés pendant 10 min à température ambiante en présence de tampon.
La capture non spécifique est déterminée par addition de 10 mM de glycine au groupe témoin.
La réaction est arrêtée par filtration sous vide et la radioactivité est estimée par scintillation solide par comptage sur un compteur Microbeta Tri-lux™.
Un inhibiteur de la capture de la [$^{14}$C]glycine diminuera la quantité de radioligand incorporée dans chaque homogénat.
L'activité du composé est évaluée par sa $DE_{50}$, dose efficace qui inhibe 50% de la capture de la [$^{14}$C]glycine par rapport au groupe témoin.
Les meilleurs composés de l'invention ont, dans ce test, une $DE_{50}$ de 1 à 20 mg/kg, par voie intrapéritonéale ou par voie orale.

Les résultats des essais effectués sur les composés de l'invention de configuration (1*S*,2*S*) et leurs racémates thréo de configuration (1*R*,2*R* ;1*S*,2*S*) dans la formule générale (I) desquels $R_2$ représente un ou plusieurs atomes d'halogènes montrent qu'ils sont des inhibiteurs du transporteur de la glycine glyt1 présents dans le cerveau, et cela in vitro et *ex vivo.*

Ces résultats suggèrent que les composés de l'invention peuvent être utilisés pour le traitement des troubles comportementaux associés à la démence, des psychoses, en particulier de la schizophrénie (forme déficitaire et forme productive) et des symptômes extrapyramidaux aigus ou chroniques induits par les neuroleptiques, pour le traitement des diverses formes d'anxiété, des attaques de panique, des phobies, des troubles obsessionnels compulsifs, pour le traitement des différentes formes de dépression, y compris la dépression psychotique, pour le traitement des troubles dus à l'abus ou au sevrage d'alcool, des troubles du comportement sexuel, des troubles de la prise de nourriture, et pour le traitement de la migraine.

Les résultats des essais effectués sur les composés de l'invention de configuration (1*R*,2*R*) et leurs racémates de configuration (1*R*,2*R*;1*S*,2*S*) dans la formule générale (I) desquels $R_2$ représente à la fois un atome d'halogène et un groupe amino $NR_6R_7$ montrent qu'ils sont des inhibiteurs du transporteur des la glycine glyt2, présent majoritairement dans la moelle épinière, et cela *in vitro* et *ex vivo.*

Ces résultats suggèrent que les composés de l'invention peuvent être utilisés pour le traitement des contractures musculaires douloureuses en rhumatologie et en pathologie rachidienne aiguë, pour le traitement des contractures spastiques d'origine médullaire ou cérébrale, pour le traitement symptomatique des douleurs aiguës et subaiguës d'intensité légère à modérée, pour le traitement des douleurs intenses et/ou chroniques, des douleurs neurogènes et algies rebelles, pour le traitement de la maladie de Parkinson et des symptômes parkinsoniens d'origine neurodégénérative ou induits par des neuroleptiques, pour le traitement des épilepsies généralisées primaires et secondaires, partielles à symptomatologie simple ou complexe, des formes mixtes et autres syndromes épileptiques en complément d'un autre traitement antiépileptique, ou en monothérapie, pour le traitement des apnées du sommeil, et pour la neuroprotection.

C'est pourquoi la présente invention a également pour objet des compositions pharmaceutiques contenant

une dose efficace d'au moins un composé selon l'invention, à l'état de base ou de sel ou de solvat pharmaceutiquement acceptable, et en mélange, le cas échéant, avec des excipients convenables.

Lesdits excipients sont choisis selon la forme pharmaceutique et le mode d'administration souhaité.

Les compositions pharmaceutiques selon l'invention peuvent ainsi être destinées à l'administration orale, sublinguale, sous-cutanée, intramusculaire, intraveineuse, topique, intratrachéale, intranasale, transdermique, rectale, intraocculaire.

Les formes unitaires d'administration peuvent être, par exemple, des comprimés, des gélules, des granules, des poudres, des solutions ou suspensions orales ou injectables, des timbres transdermiques ("patch"), des suppositoires. Pour l'administration topique on peut envisager des pommades, lotions et collyres.
Lesdites formes unitaires sont dosées pour permettre une administration journalière de 0,01 à 20 mg de principe actif par kg de poids corporel, selon la forme galénique.

Pour préparer des comprimés on ajoute au principe actif, micronisé ou non, un véhicule pharmaceutique qui peut être composé de diluants, comme par exemple le lactose, la cellulose microcristalline, l'amidon, et des adjuvants de formulation comme des liants, (polyvinylpyrrolidone, hydroxypropylméthylcellulose, etc), des agents d'écoulement comme la silice, des lubrifiants comme le stéarate de magnésium, l'acide stéarique, le tribehenate de glycerol, le stéarylfumarate de sodium. Des agents mouillants ou tensioactifs tels que le laurylsulfate de sodium peuvent aussi être ajoutés.
Les techniques de réalisation peuvent être la compression directe, la granulation sèche, la granulation humide ou la fusion à chaud.
Les comprimés peuvent être nus, dragéifiés, par exemple par du saccharose, ou enrobés avec divers polymères ou autres matières appropriées. Il peuvent être conçus pour permettre une libération rapide, retardée ou prolongée du principe actif grâce à des matrices polymères ou à des polymères spécifiques utilisés dans l'enrobage.

Pour préparer des gélules on mélange le principe actif avec des véhicules pharmaceutiques secs (simple mélange, granulation sèche ou humide, ou fusion à chaud), liquides ou semi-solides.
Les gélules peuvent être dures ou molles, pelliculées ou non, de manière à avoir une activité rapide, prolongée ou retardée (par exemple pour une forme entérique).

Une composition sous forme de sirop ou d'élixir ou pour l'administration sous forme de gouttes peut contenir le principe actif conjointement à un édulcorant, de préférence acalorique, du méthylparaben ou du propylparaben comme antiseptique, un agent de sapidité et un colorant.

Les poudres et granules dispersibles dans de l'eau peuvent contenir le principe actif en mélange avec des agents de dispersion ou des agents mouillants, ou des agents dispersants comme la polyvinylpyrrolidone, de même qu'avec des édulcorants et des agents correcteurs de goût.

Pour l'administration rectale, on recourt à des suppositoires préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles injectables contenant des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs, ou bien avec une matrice polymère ou avec une cyclodextrine (timbres transdermiques, formes à libération prolongée).

Les compositions topiques selon l'invention comprennent un milieu compatible avec la peau. Elles peuvent se présenter notamment sous forme de solutions aqueuses, alcooliques ou hydroalcooliques, de gels, d'émulsions eau-dans-huile ou huile-dans-eau ayant l'aspect d'une crème ou d'un gel, de microémulsions, d'aérosols, ou encore sous forme de dispersions vésiculaires contenant des lipides ioniques et/ou non ioniques. Ces formes galéniques sont préparées selon les méthodes usuelles des domaines considérés.

Enfin, les compositions pharmaceutiques selon l'invention peuvent contenir, à côté d'un composé de formule générale (I), d'autres principes actifs qui peuvent être utiles dans le traitement des troubles et maladies indiqués ci-dessus.

## Revendications

1. Composé, sous forme d'isomère optique pur (1*R*,2*R*) ou (1*S*,2*S*) ou sous forme de diastéréoisomère thréo, répondant à la formule générale (I)

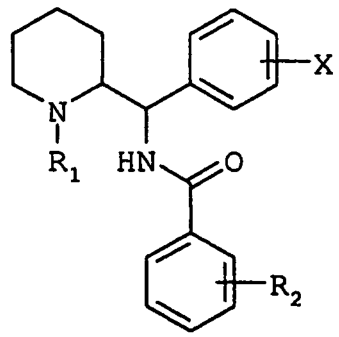

(I)

dans laquelle

$R_1$ représente soit un atome d'hydrogène, soit un groupe $(C_1-C_7)$alkyle linéaire ou ramifié éventuellement substitué par un ou plusieurs atomes de fluor, soit un groupe $(C_3-C_7)$cycloalkyl$(C_1-C_3)$alkyle, soit un groupe phényl$(C_1-C_3)$alkyle éventuellement substitué par un ou deux groupes méthoxy, soit un groupe $(C_2-C_4)$alcényle, soit un groupe $(C_2-C_4)$alcynyle,
X représente un atome d'hydrogène ou un ou plusieurs substituants choisis parmi les atomes d'halogènes et les groupes trifluorométhyle, $(C_1-C_4)$ alkyle linéaire ou ramifié et $(C_1-C_9)$ alcoxy,
$R_2$ représente un ou plusieurs substituants choisis parmi les atomes d'halogènes, parmi les groupe de formule générale $OR_3$ dans laquelle $R_3$ représente un atome d'hydrogène, un groupe $(C_1-C_4)$ alkyle, un groupe phényl $(C_1-C_3)$alkyle, ou un groupe de formule générale $(CH_2)_n-NR_4R_5$ dans laquelle n représente le nombre 2, 3 ou 4 et $R_4$ et $R_5$ représentent chacun, indépendamment l'un de l'autre, un atome d'hydrogène ou un groupe $(C_1-C_4)$ alkyle ou forment, avec l'atome d'azote qui les porte, un cycle pyrrolidine, pipéridine ou morpholine, et parmi les groupes amino de formule générale $NR_6R_7$ dans laquelle $R_6$ et $R_7$ représentent chacun, indépendamment l'un de l'autre, un atome d'hydrogène ou un groupe $(C_1-C_4)$alkyle, un groupe phényle ou un groupe phényl $(C_1-C_3)$alkyle, ou forment, avec l'atome d'azote qui les porte, un cycle pyrrolidine, pipéridine ou morpholine,

à l'état de base libre ou de sel d'addition à un acide.

2. Composé selon la revendication 1, **caractérisé en ce qu'**il est de configuration (1*S*,2*S*), avec $R_2$ représentant un ou plusieurs atomes d'halogènes.

3. Composé selon la revendication 1, **caractérisé en ce qu'**il est de configuration (1*R*,2*R*) avec $R_2$ représentant un ou plusieurs atomes d'halogènes et un groupe amino de formule générale $NR_6R_7$.

4. Médicament **caractérisé en ce qu'**il consiste en un composé selon l'une des revendications 1 à 3.

5. Composition pharmaceutique, **caractérisée en ce qu'**elle contient un composé selon l'une des revendications 1 à 3, associé à un excipient pharmaceutique.

6. Composé selon la revendication 1, **caractérisé en ce qu'**il est choisi parmi les composés suivants :

2,3-dichloro-*N*-[(1S)-[(2S)-1-méthylpipéridin-2-yl]phénylméthyl]benzamide ;
4-amino-3,5-dichloro-*N*-[(1*R*)-[(2*R*)-1-méthylpipéridin-2-yl]phénylméthyl]benzamide ;
3,5-dichloro-*N*-[(1-méthylpipéridin-2-yl)phénylméthyl]-4-(pyrrolidin-1-yl)benzamide ;
2,3-dichloro-*N*-[(*S*)-phényl[(2*S*)-pipéridin-2-yl]méthyl]benzamide.

## Claims

1. Compound, in the form of a pure optical isomer (1*R*,2*R*) or (1*S*,2*S*) or in the form of a threo diastereoisomer, corresponding to general formula (I)

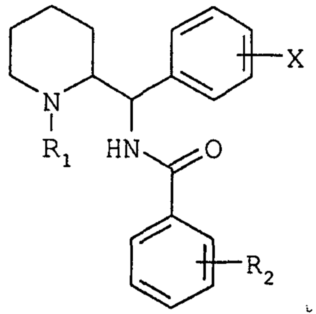

(I)

in which

$R_1$ represents either a hydrogen atom, or a linear or branched ($C_1$-$C_7$) alkyl group optionally substituted with one or more fluorine atoms, or a ($C_3$-$C_7$) cycloalkyl ($C_1$-$C_3$) alkyl group, or a phenyl ($C_1$-$C_3$)alkyl group optionally substituted with one or two methoxy groups, or a ($C_2$-$C_4$) alkenyl group, or a ($C_2$-$C_4$) alkynyl group,
X represents a hydrogen atom or one or more substituents chosen from halogen atoms and trifluoromethyl, linear or branched ($C_1$-$C_4$)alkyl and ($C_1$-$C_4$) alkoxy groups,
$R_2$ represents one or more substituents chosen from halogen atoms, from the groups of general formula $OR_3$ in which $R_3$ represents a hydrogen atom, a ($C_1$-$C_4$)alkyl group, a phenyl ($C_1$-$C_3$) alkyl group, or a group of general formula $(CH_2)_n$-$NR_4R_5$ in which n represents the number 2, 3 or 4 and $R_4$ and $R_5$ each represent, independently of each other, a hydrogen atom or a ($C_1$-$C_4$) alkyl group or form, with the nitrogen atom carrying them, a pyrrolidine, piperidine or morpholine ring, and from the amino groups of general formula $NR_6R_7$ in which $R_6$ and $R_7$ each represent, independently of each other, a hydrogen atom or a ($C_1$-$C_4$) alkyl group, a phenyl group or a phenyl($C_1$-$C_3$)alkyl group, or form, with the nitrogen atom carrying them, a pyrrolidine, piperidine or morpholine ring,

in the form of a free base or of an addition salt with an acid.

**2.** Compound according to Claim 1, **characterized in that** it has the configuration *(1S, 2S),* with $R_2$ representing one or more halogen atoms.

**3.** Compound according to Claim 1, **characterized in that** it has the configuration (1*R*, 2*R*) with $R_2$ representing one or more halogen atoms and an amino group of general formula $NR_6R_7$.

**4.** Medicament, **characterized in that** it consists of a compound according to one of Claims 1 to 3.

**5.** Pharmaceutical composition, **characterized in that** it contains a compound according to one of Claims 1 to 3, combined with a pharmaceutical excipient.

**6.** Compound according to Claim 1, **characterized in that** it is chosen from the following compounds:

2,3-dichloro-*N*-[(1*S*)-[(2*S*)-1-methylpiperidin-2-yl] phenylmethyl] benzamide;
4-amino-3,5-dichloro-*N*-[(1*R*)-[(2*R*)-1-methylpiperidin-2-yl]phenylmethyl]benzamide;
3,5-dichloro-*N*-[(1-methylpiperidin-2-yl)phenylmethyl]-4-(pyrrolidin-1-yl)benzamide;
2,3-dichloro-*N*-[(*S*)-phenyl[(2*S*)-piperidin-2-yl] methyl] benzamide.

## Patentansprüche

**1.** Verbindung, in Form eines reinen optischen Isomers (1*R*, 2*R*) oder (1*S, 2S*), oder in Form eines Threo-Diastereoisomers, mit der allgemeinen Formel (I)

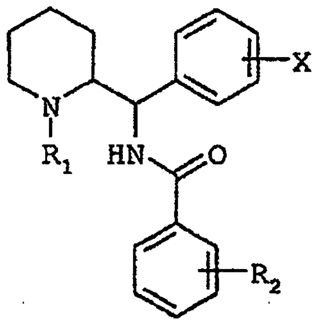

(I)

worin

$R_1$ entweder für ein Wasserstoffatom oder eine lineare oder verzweigte $C_1$-$C_7$-Alkylgruppe, die eventuell durch ein oder mehrere Fluoratome substituiert ist, oder eine $C_3$-$C_7$-Cycloalkyl-$C_1$-$C_3$-alkylgruppe, oder eine $C_1$-$C_3$-Alkylphenylgruppe, die eventuell durch eine oder zwei Methoxygruppen substituiert ist, oder eine $C_2$-$C_4$-Alkenylgruppe, oder eine $C_2$-$C_4$-Alkinylgruppe steht;
X für ein Wasserstoffatom oder einen oder mehrere Substituenten steht, ausgewählt aus den Halogenatomen und Trifluormethyl-, linearen oder verzweigten $C_1$-$C_4$-Alkyl- oder $C_1$-$C_4$-Alkoxygruppen,
$R_2$ für einen oder mehrere Substituenten steht, ausgewählt aus den Halogenatomen, aus den Gruppen der allgemeinen Formel $OR_3$, worin $R_3$ für ein Wasserstoffatom, eine $C_1$-$C_4$-Alkylgruppe, eine $C_1$-$C_3$-Alkylphenylgruppe, oder eine Gruppe der allgemeinen Formel $(CH_2)_n$-$NR_4R_5$ steht, worin n für die Zahl 2, 3 oder 4 steht, und $R_4$ und $R_5$ jeweils unabhängig voneinander für ein Wasserstoffatom oder eine $C_1$-$C_4$-Alkylgruppe stehen, oder mit dem Stickstoffatom, das sie trägt, einen Pyrrolidin-, Piperidin- oder Morpholinring bilden, und aus den Aminogruppen der allgemeinen Formel $NR_6R_7$, worin $R_6$ und $R_7$ jeweils unabhängig voneinander für ein Wasserstoffatom oder eine $C_1$-$C_4$-Alkylgruppe, eine Phenylgruppe oder eine $C_1$-$C_3$-Alkylphenylgruppe stehen, oder mit dem Stickstoffatom, das sie trägt, einen Pyrrolidin-, Piperidin- oder Morpholinring bilden,

im Zustand einer freien Base oder eines Säureadditionssalzes.

**2.** Verbindung nach Anspruch 1, **dadurch gekennzeichnet, dass** sie die Konfiguration (1*S*, 2*S*) aufweist, wobei $R_2$ für ein oder mehrere Halogenatome steht.

**3.** Verbindung nach Anspruch 1, **dadurch gekennzeichnet, dass** sie die Konfiguration (1*R*, 2R) aufweist, wobei $R_2$ für ein oder mehrere Halogenatome und die Aminogruppen der allgemeinen Formel $NR_6R_7$ steht.

**4.** Medikament, **dadurch gekennzeichnet, dass** es aus einer Verbindung nach einem der Ansprüche 1 bis 3 besteht.

**5.** Pharmazeutische Zusammensetzung **dadurch gekennzeichnet, dass** sie eine Verbindung nach einem der Ansprüche 1 bis 3 enthält, die mit einem pharmazeutischen Hilfsstoff kombiniert ist.

**6.** Verbindung nach Anspruch 1, **dadurch gekennzeichnet, dass** sie ausgewählt ist aus den folgenden Verbindungen

2,3-Dichlor-*N*-[(1*S*)-[(2*S*)-1-methylpiperidin-2-yl]phenylmethyl]benzamid;
4-Amino-3,5-dichlor-*N*-[(1*R*)-[(2*R*)-1-methylpiperidin-2-yl]phenylmethyl]benzamid;
3,5-Dichlor-*N*-[(1-methylpiperidin-2-yl)phenylmethyl]-4-(pyrrolidin-1-yl)benzamid;
2,3-Dichlor-*N*-[(*S*)-phenyl[(2*S*)-piperidin-2-yl]methyl]benzamid.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 5254569 A
- EP 0499995 A
- WO 9945011 A
- EP 0366006 A
- US 2928835 A
- EP 0556672 A
- US 3801636 A


### Littérature non-brevet citée dans la description


- *Eur. J. Med. Chem.,* 2000, vol. 35, 979-988
- *J. Med. Chem.,* 1998, vol. 41, 591-601
- *Tetrahedron Lett,* 1996, vol. 57 (33), 5905-5908
- *Chem. Pharm. Bull,* 1984, vol. 32 (12), 4893-4906
- *Synthesis,* 1976, 593-595
- *J. Chem. Soc.,* 1927, 25
- *Chem. Pharm. Bull.,* 1992, 1789-1792
- *Aust. J. Chem.,* 1984, 1938-1950
- *J. O. C.,* 1980, 527